(19) Europäisches Patentamt
European Patent Office
Office européen des brevets

(11) Veröffentlichungsnummer : **0 039 034**
**B1**

(12) # EUROPÄISCHE PATENTSCHRIFT

(45) Veröffentlichungstag der Patentschrift : **28.12.83**

(51) Int. Cl.³ : **C 07 C119/06**, A 61 K 7/13

(21) Anmeldenummer : **81102992.5**

(22) Anmeldetag : **18.04.81**

(54) **Kupplerkomponenten für Oxidationshaarfarben und deren Verwendung, sowie diese enthaltende Haarfärbemittel.**

(30) Priorität : **30.04.80 CH 3331/80**
**02.05.80 DE 3016906**

(43) Veröffentlichungstag der Anmeldung :
**04.11.81 Patentblatt 81/44**

(45) Bekanntmachung des Hinweises auf die Patenterteilung : **28.12.83 Patentblatt 83/52**

(84) Benannte Vertragsstaaten :
**AT BE CH DE FR GB IT LI LU NL SE**

(56) Entgegenhaltungen :
**DE-A- 2 509 096**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Rose, David, Dr.**
**Holbeinweg 7**
**D-4010 Hilden (DE)**
Erfinder : **Maak, Norbert, Dr.**
**Liebigstrasse 18**
**D-4040 Neuss (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 039 034 B1

# Kupplerkomponenten für Oxidationshaarfarben und deren Verwendung, sowie diese enthaltende Haarfärbemittel

Gegenstand der Erfindung sind 2,4-Dichlor-3-alkyliden-aminophenole der allgemeinen Formel

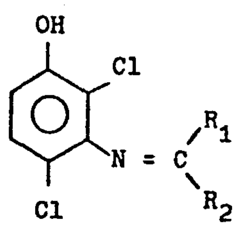

in der $R_1$ Wasserstoff oder einen Alkylrest mit 1-Kohlenstoffatomen und $R_2$ einen Alkyl- oder Arylrest darstellen, mit der Maßgabe, daß $R_2$ nur dann ein Arylrest sein kann, wenn $R_1$ Wasserstoff ist, sowie deren Herstellung. Vorzugsweise bedeutet $R_1$ und $R_2$, welche gleich oder verschieden sein können, einen Alkylrest mit 1-4 Kohlenstoffatomen.

Die Herstellung der 2,4-Dichlor-3-alkylidenaminophenole erfolgt durch längeres Erhitzen von 2,4-Dichlor-3-aminophenol mit Verbindung der allgemeinen Formel

$$R_1\,CO\,R_2,$$

wobei $R_1$ und $R_2$ die oben genannte Bedeutung haben.

Verbindungen der Formel $R_1\,CO\,R_2$ werden vorzugsweise im Überschuß angewendet, wobei diese gleichzeitig als Lösungsmittel dienen. Das Verfahren kann auch in Gegenwart anderer Lösungsmittel, wie Alkohole oder chlorierter Kohlenwasserstoffe, durchgeführt werden. Man kocht kurze Zeit unter Rückfluß oder läßt einige Stunden bei 0-50 °C reagieren.

Die Umsetzung wird zweckmäßig in Gegenwart wasserabspaltungsbegünstigender Katalysatoren durchgeführt. Solche sind vorteilhafterweise Mineralsäuren, wie HCl, $H_2SO_4$, sowie Toluolsulfonsäure u. a. Vorzugsweise wird HCl angewendet.

Weitere Gegenstände der Erfindung sind die Verwendung der 2,4-Dichlor-3-alkylidenaminophenole als solche oder in Gestalt ihrer Salze mit anorganischen oder organischen Säuren als Kupplerkomponenten in Oxidationshaarfarben sowie Haarfärbemittel, die die neuen 2,4-Dichlor-3-alkylidenaminophenole enthalten.

Für das Färben von Haaren spielen die sogenannten Oxidationsfarben, die durch oxidative Kupplung einer Entwicklerkomponente mit einer Kupplerkomponente entstehen, wegen ihrer intensiven Farben und sehr guten Echtheitseigenschaften eine bevorzugte Rolle. Als Entwicklersubstanzen werden üblicherweise Stickstoffbasen wie p-Phenylendiaminderivate, Diaminopyridine, 4-Aminopyrazolonderivate, heterocyclische Hydrazone eingesetzt. Als sogenannte Kupplerkomponenten werden m-Phenylendiaminderivate, Phenole, Naphthole, Resorcinderivate und Pyrazolone genannt.

Aus der deutschen Offenlegungsschrift Nr. 25 09 096 sind mehrfach halogensubstituierte m-Aminophenole bekannt, die sich als Kuppler für Oxidationshaarfärbemittel eignen. Die Produkte weisen jedoch einen intensiven phenolischen Geruch auf, der bei der Herstellung und Verarbeitung zu kosmetischen Präparaten störend ist.

Gute Oxidationshaarfarbstoffkomponenten müssen in erster Linie folgende Voraussetzungen erfüllen :

Sie müssen bei der oxidativen Kupplung mit den jeweiligen Entwickler- bzw. Kupplerkomponenten die gewünschten Farbnuancen in ausreichender Intensität ausbilden. Sie müssen ferner ein ausreichendes bis sehr gutes Aufziehvermögen auf menschlichem Haar besitzen und sie sollen darüber hinaus in toxikologischer und dermatologischer Hinsicht unbedenklich sein.

Es bestand daher bei der Suche nach brauchbaren Oxidationshaarfarbstoffen die Aufgabe, geeignete Komponenten aufzufinden, die vorgenannte Voraussetzungen in optimaler Weise erfüllen.

Es wurde nun gefunden, daß man zu Oxidationshaarfarben, die den gestellten Anforderungen in besonders hohem Maße gerecht werden, gelangt, wenn man als Kupplerkomponenten 2,4-Dichlor-3-alkylidenaminophenole der allgemeinen Formel

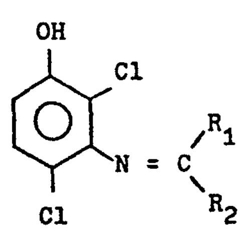

in der $R_1$ Wasserstoff oder einen Alkylrest mit 1-6 Kohlenstoffatomen und $R_2$ einen Alkyl- oder Arylrest darstellen, mit der Maßgabe, daß $R_2$ nur dann ein Arylrest sein kann, wenn $R_1$ Wasserstoff ist, sowie deren Salze mit anorganischen oder organischen Säuren in Kombination mit üblichen Entwicklersubstanzen, verwendet.

Haarfärbemittel auf Basis von Oxidationsfarben mit einem Gehalt an 2,4-Dichlor-3-alkylidenaminophenolen der allgemeinen Formel

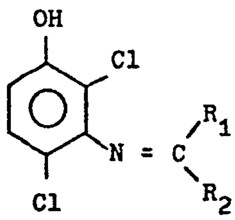

in der $R_1$ und $R_2$ die vorgenannte Bedeutung haben, sowie deren Salzen mit anorganischen oder organischen Säuren als Kupplerkomponenten und den in Oxidationshaarfarben üblichen Entwicklersubstanzen stellen demnach wertvolle Kompositionen auf dem Gebiet der Oxidationshaarfarben dar.

Bei ihrem Einsatz als Kupperlkomponenten liefern die erfindungsgemäßen Verbindungen mit den im allgemeinen für die Oxidationshaarfärbung verwendeten Entwicklersubstanzen sehr intensive, von braun über dunkelblau bis dunkelviolett reichende Farbtöne und stellen somit eine wesentliche Bereicherung der oxidativen Haarfärbemöglichkeiten dar. Darüber hinaus zeichnen sich die erfindungsgemäßen 2,4-Dichlor-3-alkylidenaminophenole durch sehr gute Echtheitseigenschaften der damit erzielten Färbungen, durch eine gute Löslichkeit in Wasser, eine gute Lagerstabilität und toxikologische sowie dermatologische Unbedenklichkeit aus.

Die erfindungsgemäß als Kupplerkomponenten zu verwendenden 2,4-Dichlor-3-alkylidenaminophenole können entweder als solche oder in Form ihrer Salze mit anorganischen oder organischen Säuren wie zum Beispiel als Chloride, Sulfate, Phosphate, Acetate, Propionate, Lactate, Citrate eingesetzt werden.

Als erfindungsgemäß einzusetzende Kupplerkomponenten sind zum Beispiel 2,4-Dichlor-3-(methyl-3'-butyliden-2)-aminophenol, 2,4-Dichlor-3-(isopropyliden)-iminophenol, 2,4-Dichlor-3-benzylidenaminophenol zu nennen.

Als Beispiel für in den erfindungsgemäßen Haarfärbemitteln einzusetzende Entwicklerkomponenten sind primäre aromatische Amine mit einer weiteren in p-Stellung befindlichen funktionellen Gruppe wie p-Phenylendiamin, p-Toluylendiamin, p-Aminophenol, N-Methyl-p-phenylendiamin, N,N-Dimethyl-p-phenylendiamin, N-Ethyl-N-hydroxyethyl-p-phenylendiamin, Chlor-p-phenylendiamin, N,N-Bis-hydroxyethylamino-p-phenylendiamin, Methoxy-p-phenylendiamin, 2-Chlor-, 2,6-Dichlor-p-phenylendiamin, 2-Chlor-6-brom-p-phenylendiamin, 2-Chlor-6-methyl-p-phenylendiamin, 6-Methoxy-3-methyl-p-phenylendiamin, andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxyalkylrest mit 1-4 Kohlenstoffatomen darstellt, ferner Diaminopyridinderivate, heterocyclische Hydrazonderivate wie 1-Methyl-pyrrolidon-(2)-hydrazon, 4-Aminopyrazolonderivate wie 4-Amino-1-phenyl-3-carbamoylpyrazolon-5, N-Butyl-N-sulfobutyl-p-phenylendiamin, Tetraaminopyrimidine wie 2,4,5,6-Tetraaminopyrimidin, 4,5-Diamino-2,6-bismethylaminopyrimidin, 2,5-Diamino-4-diethylamino-6-methylaminopyrimidin, 2,4,5-Triamino-6-dimethylaminopyrimidin, 2,4,5-Triamino-6-piperidinopyrimidin, 2,4,5-Triamino-6-anilino-pyrimidin, 2,4,5-Triamino-6-morpholinopyrimidin, 2,4,5-Triamino-6-β-hydroxy-ethylaminopyrimidin anzuführen.

Die erfindungsgemäßen Kupplerkomponenten liefern neben anderen Farbnuancen mit entsprechenden Entwicklersubstanzen dunkelblaue, besonders intensive Haarfärbungen, die sich durch außerordentliche Lichtechtheiten auszeichnen. Sie sind daher auch als Nuancierkomponente zur Erzielung mölgichst kräftiger und den natürlichen Haarfarbnuancen weitgehend entsprechender Farbtöne von besonderer Wichtigkeit, da sich bei der Erstellung natürlicher Farbnuancen unter Zuhilfenahme von Blaukupplerkomponenten häufig Schwierigkeiten ergeben.

In Haarfärbemitteln werden die erfindungsgemäßen Kupplerkomponenten im allgemeinen in etwa molaren Mengen, bezogen auf die verwendeten Entwicklersubstanzen, eingesetzt. Wenn sich auch der molare Einsatz als zweckmäßig erweist, so ist es jedoch nicht nachteilig, wenn die Kupplerkomponente in einem gewissen Überschuß oder Unterschuß zum Einsatz gelangt.

Es ist ferner nicht erforderlich, daß die Entwicklerkomponente und die Kupplersubstanz einheitliche Produkte darstellen, vielmehr können sowohl die Entwicklerkomponente Gemische der erfindungsgemäß zu verwendenden Entwicklerverbindungen, als auch die Kupplersubstanz Gemische der erfindungsgemäß einzusetzenden 2,4-Dichlor-3-alkylideniminophenole gegebenenfalls im Gemisch mit weiteren üblichen Kupplersubstanzen darstellen.

Darüber hinaus können die erfindungsgemäßen Haarfärbemittel gegebenenfalls übliche direktziehende Farbstoffe im Gemisch enthalten, falls dies zur Erzielung gewisser Farbnuancen erforderlich ist.

Die oxidative Kupplung, das heißt die Entwicklung der Färbung, kann grundsätzlich wie bei anderen Oxidationshaarfarbstoffen auch, durch Luftsauerstoff erfolgen. Zweckmäßigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxiddisulfat in Betracht.

Die erfindungsgemäßen Haarfärbemittel werden für den Einsatz in entsprechende kosmetische Zubereitungen wie Cremes, Emulsione, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwicklerkombination beträgt 0,2 bis 5 Gewichtsprozent, vorzugsweise 1 bis 3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind zum Beispiel Netz- oder Emulgiermittel vom anionischen oder nichtionogenen Typ wie Alkylbenzolsulfonate, Fettalkoholsulfate, Alkylsulfonate, Fettsäurealkanolamide, Anlagerungsprodukte von Ethylenoxid an Fettalkohole, Verdickungsmittel wie Methylcellulose, Stärke, höhere Fettalkohole, Paraffinöl, Fettsäuren, ferner Parfümöle und Haarpflegemittel wie Pantothensäure und Cholesterin zu nennen. Die genannten Zusatzstoffe werden dabei in den für diese Zwecke üblichen Mengen eingesetzt, wie zum Beispiel Netz- und Emulgiermittel in Konzentrationen von 0,5 bis 30 Gewichtsprozent und Verdickungsmittel in Konzentrationen von 0,1 bis 25 Gewichtsprozent, jeweils bezogen auf die gesamte Zubereitung.

Die Anwendung der erfindungsgemäßen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8-10 erfolgen. Die Anwendungstemperaturen bewegen sich dabei im Bereich von 15 bis 40 °C. Nach einer Einwirkungsdauer von ca. 30 Minuten wird das Haarfärbemittel vom zu färbenden Haar durch Spülen entfernt. Hernach wird das Haar mit einem milden Shampoo nachgewaschen und getrocknet.

Die mit den erfindungsgemäßen Haarfärbemitteln erzielbaren Färbungen, insbesondere auch die Blautöne zeigen unter Einsatz unterschiedlicher Entwickler- und Kupplerkomponenten besonders intensive Farbnuancen. Die erzielten Färbungen haben gute Licht-, Wasch- und Reibechtheitseigenschaften und lassen sich leicht mit Reduktionsmitteln wieder abziehen.

Die nachfolgenden Beispiele sollen den Erfindungsgegenstand näher erläutern, ohne ihn jedoch hierauf zu beschränken.

## Beispiele

### Beispiel 1

2,4-Dichlor-3-(methyl-3'-butyliden-2)-aminophenolhydrochlorid

5 g 2,4-Dichlor-3-aminophenol-hydrochlorid (hergestellt entsprechend den Angaben in der deutschen Offenlegungsschrift 2 509 096) wurden in 50 ml Methylisobutylketon eine halbe Stunde lang unter Rückfluß gekocht. Nach dem Abkühlen wurde das Produkt abgesaugt und getrocknet. Das erhaltene 2,4-Dichlor-3-(methyl-3'-butyliden-2)-aminophenol-hydrochlorid wies folgendes IR-Absorptionsspektrum auf :

IR-Spektrum (KBr) $cm^{-1}$ : 1 650, 1 620, 1 520, 1 475, 1 435, 1 370, 1 315, 1 260, 1 215, 1 180, 1 090, 1 045, 1 010, 980, 970, 825, 815, 790, 785, 760, 690.

### Beispiel 2

2,4-Dichlor-3-(isopropyliden)-aminophenol-hydrochlorid

Entsprechend den Angaben bei Beispiel 1 wurde 2,4-Dichlor-3-aminophenol-hydrochlorid mit Aceton umgesetzt. Das erhaltene erfindungsgemäße Reaktionsprodukt besitzt folgende Kenndaten :

IR-Spektrum (KBr) $cm^{-1}$ : 1 652, 1 598, 1 570, 1 480, 1 438, 1 370, 1 315, 1 260, 1 238, 1 225, 1 218, 1 176, 1 112, 1 085, 1 010, 980, 930, 870, 835, 805, 762, 730, 710, 675, 655.

### Beispiel 3

2,4-Dichlor-3-benzyliden-aminophenol-hydrochlorid

Analog den Angaben bei Beispiel 1 wurde 2,4-Dichlor-3-aminophenol-hydrochlorid mit Benzaldehyd umgesetzt. Das erfindungsgemäße Reaktionsprodukt besitzt folgende Kenndaten :

IR-Spektrum (KBr) $cm^{-1}$ : 1 692, 1 620, 1 598, 1 582, 1 515, 1 480, 1 450, 1 430, 1 310, 1 205, 1 160, 1 075, 1 030, 930, 905, 890, 882, 830, 805, 745, 700, 685, 650.

Die vorgenannten, in ihrer Herstellung in den Beispielen 1, 2 und 3 beschriebenen 2,4-Dichlor-3-alkylidenaminophenole wurden als Kupplerkomponenten in den folgenden Versuchen eingesetzt.
Als Entwicklerkomponenten dienten folgende Substanzen :

E 1 : p-Toluylendiamin
E 2 : 2,4,5,6-Tetraaminopyrimidin
E 3 : 2-Piperidino-4,5,6-Triaminopyrimidin
E 4 : 2-Methylamino-4,5,6-Triaminopyrimidin
E 5 : 2-Morpholino-4,5,6-triaminopyrimidin
E 6 : p-Phenylendiamin
E 7 : 2-Chlor-p-phenylendiamin
E 8 : p-Aminophenol
E 9 : N-Methyl-p-phenylendiamin
E 10 : N-Ethyl-N-β-hydroxyethyl-p-phenylendiamin
E 11 : N,N-Bis-(β-hydroxymethyl)-amino-p-phenylendiamin.

Die erfindungsgemäßen Haarfärbemittel wurden in Form einer Cremeemulsion eingesetzt. Dabei wurden in eine Emulsion aus

10 Gewichtsteilen Fettalkoholen der Kettenlänge $C_{12}$-$C_{18}$,
10 Gewichtsteilen Fettalkoholsulfat (Natriumsalz) der Kettenlänge $C_{12}$-$C_{18}$ und
75 Gewichtsteilen Wasser

jeweils 0,01 Mol der in der nachstehenden Tabelle aufgeführten Entwicklersubstanzen und Kupplersubstanzen eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde mit 1 %iger Wasserstoffperoxidlösung als Oxidationsmittel durchgeführt, wobei zu 100 Gewichtsteilen der Emulsion 10 Gewichtsteile Wasserstoffperoxidlösung gegeben wurden. Die jeweilige Färbecreme mit Zusatz von Oxidationsmitteln wurde auf zu 90 % ergrautes, nicht besonders vorbehandeltes Menschenhaar aufgetragen und dort 30 Minuten belassen. Nach Beendigung des Färbeprozesses wurde das Haar mit einem üblichen Haarwaschmittel ausgewaschen und anschließend getrocknet. Die dabei erhaltenen Färbungen sind nachstehender Tabelle 1 zu entnehmen.

Tabelle 1

| Versuch Nr. | Entwickler | Kuppler gemäß Beispiel | Erhaltener Farbton mit 1 %iger $H_2O_2$-Lösung |
|---|---|---|---|
| 1 | E 1 | 1 | dunkelviolett |
| 2 | E 1 | 2 | dunkelblau |
| 3 | E 2 | 2 | blau |
| 4 | E 3 | 2 | oliv |
| 5 | E 4 | 2 | blaugrau |
| 6 | E 5 | 2 | grautürkis |
| 7 | E 6 | 2 | dunkelviolett |
| 8 | E 7 | 2 | aubergine |
| 9 | E 8 | 2 | braun |
| 10 | E 9 | 2 | schwarzblau |
| 11 | E 10 | 2 | schwarzblau |
| 12 | E 11 | 2 | dunkelblau |
| 13 | E 1 | 3 | graubraun |

**Ansprüche** (für die Vertragsstaaten : BE, CH, DE, FR, GB, IT, LI, LU, NL, SE)

1. 2,4 Dichlor-3-alkylidenaminophenole der allgemeinen Formel

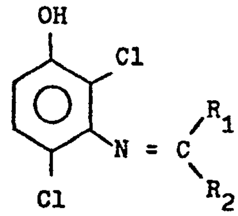

in der $R_1$ Wasserstoff oder einen Alkylrest mit 1-6 Kohlenstoffatomen und $R_2$ einen Alkyl- oder Arylrest darstellen, mit der Maßgabe, daß $R_2$ nur dann ein Arylrest sein kann, wenn $R_1$ Wasserstoff ist.

2. 2,4-Dichlor-3-alkylidenaminophenole der allgemeinen Formel

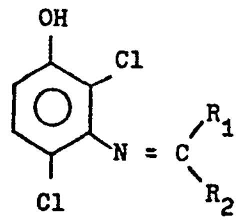

wobei $R_1$ und $R_2$ gleich oder verschieden sein können und einen Alkylrest mit 1-4 Kohlenstoffatomen bedeuten.

3. Verwendung von 2,4-Dichlor-3-alkylidenaminophenolen, gemäß Anspruch 1, sowie deren Salzen mit anorganischen oder organischen Säuren als Kupplerkomponenten in Oxidationshaarfarbstoffen.

4. Verwendung von 2,4-Dichlor-3-alkylidenaminophenolen, gemäß Anspurch 2, sowie deren Salzen mit anorganischen oder organischen Säuren als Kupplerkomponenten in Oxidationshaarfarbstoffen.

5. Haarfärbemittel auf Basis von Oxidationsfarbstoffen, gekennzeichnet durch einen Gehalt an 2,4-Dichlor-3-alkylidenaminophenolen nach Anspruch 1, sowie deren Salzen mit anorganischen oder organischen Säuren als Kupplersubstanzen und den in Oxidationsfarben üblichen Entwicklerkomponenten.

6. Haarfärbemittel auf Basis von Oxidationsfarbstoffen, gekennzeichnet durch einen Gehalt an 2,4-Dichlor-3-alkylidenaminophenolen nach Anspruch 2, sowie deren Salzen mit anorganischen oder organischen Säuren als Kupplersubstanzen und den in Oxidationsfarben üblichen Entwicklerkomponenten.

7. Haarfärbemittel nach Anspruch 5-6, gekennzeichnet durch einen Gehalt an Entwickler-Kuppler-Kombinationen von 0,2-5 Gewichtsprozent, vorzugsweise von 1-3 Gewichtsprozent, bezogen auf das gesamte Mittel.

**Ansprüche** (für den Vertragsstaat AT)

1. Verwendung von 2,4-Dichlor-3-alkylidenaminophenolen der allgemeinen Formel I

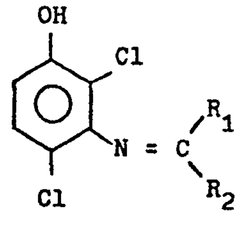

(I)

in der $R_1$ Wasserstoff oder einen Alkylrest mit 1-6 Kohlenstoffatomen und $R_2$ einen Alkyl- oder Arylrest darstellen, mit der Maßgabe, daß $R_2$ nur dann ein Arylrest sein kann, wenn $R_1$ Wasserstoff ist sowie deren Salzen mit anorganischen oder organischen Säuren als Kupplerkomponenten in Oxidationshaarfarbstoffen.

2. Verwendung von 2,4-Dichlor-3-alkylidenaminophenolen der allgemeinen Formel II

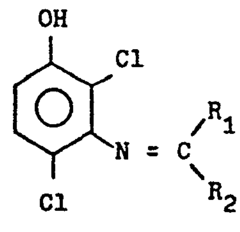

(II)

wobei $R_1$ und $R_2$ gleich oder verschieden sein können und einen Alkylrest mit 1-4 Kohlenstoffatomen bedeuten sowie deren Salzen mit anorganischen oder organischen Säuren als Kupplerkomponenten in Oxidationshaarfarbstoffen.

3. Haarfärbemittel auf Basis von Oxidationsfarbstoffen, gekennzeichnet durch einen Gehalt an 2,4-Dichlor-3-alkylidenaminophenolen der Formel I gemäß Anspruch 1 sowie deren Salzen mit anorganischen oder organischen Säuren als Kupplersubstanzen und den in Oxidationsfarben üblichen Entwicklerkomponenten.

4. Haarfärbemittel auf Basis von Oxidationsfarbstoffen, gekennzeichnet durch einen Gehalt an 2,4-Dichlor-3-alkylidenaminophenolen der Formel II gemäß Anspruch 2 sowie deren Salzen mit anorganischen oder organischen Säuren als Kupplersubstanzen und den in Oxidationsfarben üblichen Entwicklerkomponenten.

5. Haarfärbemittel nach Anspruch 3 und 4, gekennzeichnet durch einen Gehalt an Entwickler-Kuppler-Kombinationen von 0,2-5 Gew.-%, vorzugsweise von 1-3 Gew.-%, bezogen auf das gesamte Mittel.

**Claims** (for the Contracting States : BE, CH, DE, FR, GB, IT, LI, LU, NL, SE)

1. 2,4-Dichloro-3-alkylidene aminophenols corresponding to the following general formula

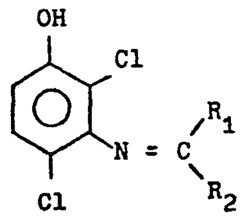

in which $R_1$ represents hydrogen or a $C_1$-$C_6$-alkyl radical and $R_2$ represents an alkyl or aryl radical with the proviso that $R_2$ can only be an aryl radical when $R_1$ is hydrogen.

2. 2,4-Dichloro-3-alkylidene aminophenols corresponding to the following general formula

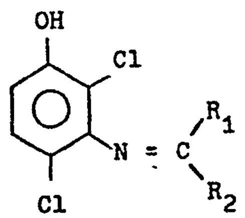

in which $R_1$ and $R_2$ may be the same or different and represent a $C_1$-$C_4$ alkyl radical.

3. The use of 2,4-dichloro-3-alkylidene aminophenols of the type claimed in Claim 1 and their salts with inorganic or organic acids as coupler components in oxidation hair dyes.

4. The use of 2,4-dichloro-3-alkylidene aminophenols of the type claimed in Claim 2 and their salts with inorganic or organic acids as coupler components in oxidation hair dyes.

5. Hair dyes based on oxidation dyes, characterized by a content of the 2,4-dichloro-3-alkylidene aminophenols claimed in Claim 1 and their salts with inorganic or organic acids as coupler substances and the developer components normally used in oxidation dyes.

6. Hair dyes based on oxidation dyes, characterized by a content of the 2,4-dichloro-3-alkylidene aminophenols claimed in Claim 2 and their salts with inorganic or organic acids as coupler substances and the developer components normally used in oxidation dyes.

7. Hair dyes as claimed in Claims 5 and 6, characterized by a content of from 0.2 to 5 % by weight and preferably from 1 to 3 % by weight, based on the dye as a whole, of developer-coupler combinations.

**Claims** (for the Contracting State AT)

1. The use of 2,4-dichloro-3-alkylidene aminophenols corresponding to the following general formula

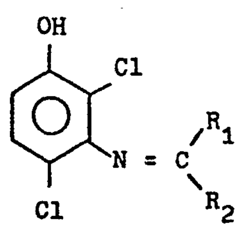

(I)

in which $R_1$ represents hydrogen or a $C_1$-$C_6$-alkyl radical and $R_2$ represents an alkyl or aryl radical with the proviso that $R_2$ can only be an aryl radical when $R_1$ is hydrogen, and their salts with inorganic or organic acids as coupler components in oxidation hair dyes.

2. The use of 2,4-dichloro-3-alkylidene aminophenols corresponding to the following general formula

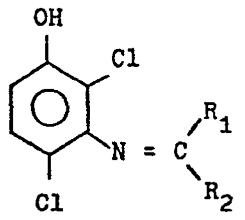

(II)

in which $R_1$ and $R_2$ may be the same or different and represent a $C_1$-$C_4$ alkyl radical and their salts with inorganic or organic acids as coupler components in oxidation hair dyes.

3. Hair dyes based on oxidation dyes, characterized by a content of the 2,4-dichloro-3-alkylidene aminophenols corresponding to formula I in Claim 1 and their salts with inorganic or organic acids as coupler substances and the developer components normally used in oxidation dyes.

4. Hair dyes based on oxidation dyes, characterized by a content of the 2,4-dichloro-3-alkylidene aminophenols corresponding to formula II in Claim 2 and their salts with inorganic or organic acids as coupler substances and the developer components normally used in oxidation dyes.

5. Hair dyes as claimed in Claims 3 and 4, characterized by a content of from 0.2 to 5 % by weight and preferably from 1 to 3 % by weight, based on the dye as a whole, of developer-coupler combinations

**Revendications** (pour les Etats contractants : BE, CH, DE, FR, GB, IT, LI, LU, NL, SE)

1. 2,4-Dichloro-3-alkylidène-aminophénols de formule générale

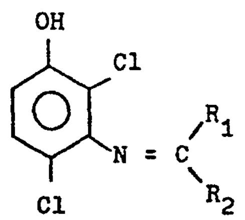

dans laquelle $R_1$ représente l'hydrogène ou un groupe alkyle en $C_1$-$C_6$ et $R_2$ représente un groupe alkyle ou aryle, étant spécifié que $R_2$ ne peut représenter un groupe aryle que lorsque $R_1$ représente l'hydrogène.

2. 2,4-Dichloro-3-alkylidène-aminophénols de formule générale

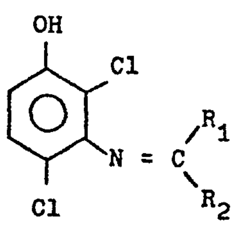

dans laquelle $R_1$ et $R_2$, identiques ou différents, représentent chacun un groupe alkyle en $C_1$-$C_4$.

3. Utilisation des 2,4-dichloro-3-alkylidène-aminophénols selon la revendication 1 et de leurs sels d'acides minéraux ou organiques en tant que copulants dans des colorants pour cheveux par oxydation.

4. Utilisation des 2,4-dichloro-3-alkylidène-aminophénols selon la revendication 2 et de leurs sels d'acides organiques ou minéraux en tant que copulants dans des colorants pour cheveux par oxydation.

5. Teinture pour cheveux à base de colorants par oxydation, caractérisée en ce qu'elle contient des 2,4-dichloro-3-alkylidène-aminophénols selon la revendication 1 ou leurs sels d'acides minéraux ou organiques en tant que copulants avec les révélateurs usuels dans les teintures par oxydation.

6. Teinture pour cheveux à base de colorant par oxydation, caractérisée en ce qu'elle contient des 2, 4-dichloro-3-alkylidène-aminophénols selon la revendication 2 ou leurs sels d'acides minéraux ou organiques en tant que copulants avec des révélateurs usuels dans les teintures par oxydation.

7. Teinture pour cheveux selon les revendications 5 et 6, caractérisée en ce qu'elle contient la combinaison copulant-révélateur en quantité de 0,2 à 5 % en poids, de préférence de 1 à 3 % en poids, par rapport au produit total.

**Revendications** (pour l'Etat contractant AT)

1. Utilisation de 2,4-dichloro-3-alkylidèneaminophénols de formule générale I

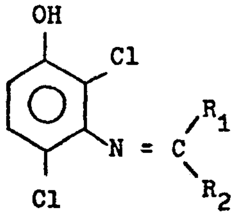

(I)

dans laquelle $R_1$ représente l'hydrogène ou un groupe alkyle en $C_1$-$C_6$ et $R_2$ représente un groupe alkyle ou aryle, avec la condition que $R_2$ ne peut être un reste aryle que si $R_1$ est l'hydrogène, et leurs sels d'acides inorganiques ou organiques comme composants copulants dans les colorants pour cheveux par oxydation.

2. Utilisation de 2,4-dichloro-3-alkylidèneaminophénols de formule générale II

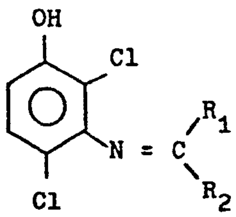

(II)

dans laquelle $R_1$ et $R_2$ peuvent être identiques ou différents et réprésentent un reste alkyle en $C_1$-$C_4$, et leurs sels d'acides inorganiques ou organiques comme composants copulants dans les colorants pour cheveux par oxydation.

3. Teinture pour cheveux à base de colorants par oxydation, caractérisée en ce qu'elle contient des 2,4-dichloro-3-alkylidèneaminophénols de formule I selon la revendication 1 et leurs sels d'acides inorganiques ou organiques comme copulants et les révélateurs habituels dans les teintures par oxydation.

4. Teinture pour cheveux à base de colorants par oxydation, caractérisée en ce qu'elle contient des 2,4-dichloro-3-alkylidèneaminophénols de formule II selon la revendication 2 et leurs sels d'acides inorganiques et organiques comme copulants et les révélateurs usuels dans les teintures par oxydation.

5. Teinture pour cheveux selon les revendications 3 et 4, caractérisée en ce qu'elle contient des combinaisons révélateur-copulant en quantité de 0,2-5 % en poids, de préférence 1-3 % en poids, par rapport au produit total.